# DEMANDE DE BREVET EUROPEEN

(11) **EP 1 997 449 A2**
(43) Date de publication de la demande: **03.12.2008**
(21) Numéro de dépôt: 08356074.8
(22) Date de dépôt: 29.05.2008
(51) Int. Cl.: A61B 17/70

(54) **Dispositif et ensemble de guidage dynamique postérieur du rachis et système de traitement du rachis comportant un tel dispositif**

(30) Priorité: 31.05.2007 FR 0703881
(71) Demandeur: PHUSIS, 38330 Saint Ismier (FR); Tornier, Alain, 38330 Saint Ismier (FR)
(72) Inventeur: Tornier, Alain, 38330 Saint Ismier (FR)
(74) Mandataire: Grand, Guillaume

(57) **Abrégé**

Ce dispositif de guidage (100) comporte deux ensembles vertébraux (110, 120) respectivement adaptés pour être fixés au côté postérieur de deux vertèbres adjacentes (1, 2) du rachis. Pour guider efficacement et de manière stable ces vertèbres en vue de reproduire une liaison intervertébrale articulaire entre elles, ces deux ensembles vertébraux définissent respectivement des surfaces d'articulation complémentaires (111 P, 121A) sensiblement sphériques, qui, lorsque les ensembles sont fixés aux vertèbres, s'étendent globalement du côté postérieur des vertèbres et glissent l'une contre l'autre, en étant centrées en un même point géométrique (C) situé dans l'espace intervertébral séparant les vertèbres, tandis que des moyens mécaniques (130) limitent l'amplitude des glissements relatifs des surfaces d'articulation.

## Description

La présente invention concerne un dispositif de guidage dynamique postérieur du rachis, destiné à être implanté le long du côté postérieur de la colonne vertébrale en vue de guider les mouvements de deux vertèbres adjacentes l'une par rapport à l'autre, en reproduisant une liaison intervertébrale articulaire. L'invention concerne également un ensemble comportant au moins deux de ces dispositifs, ainsi qu'un système de traitement du rachis comportant un tel dispositif.

L'invention s'intéresse ainsi au traitement d'un rachis dégénératif ou traumatique, plus particulièrement au niveau dorsolombaire de la colonne vertébrale, mais également au niveau cervical.

Pour traiter une instabilité intervertébrale, une première possibilité connue consiste à fusionner deux vertèbres adjacentes, ce qui revient à priver ces deux vertèbres de leur liberté de mouvements relatifs. Des montages totalement rigides sont à cet effet implantés de manière fixe le long du rachis, pour bloquer définitivement la liaison articulaire entre les deux vertèbres à fusionner. Cette intervention d'arthrodèse conduit cependant à une dégénérescence des disques adjacents sur lesquels il est souvent nécessaire d'intervenir ultérieurement.

Une autre possibilité connue de traitement consiste à intervenir à un stade plus précoce que celui nécessitant une arthrodèse et vise à implanter un dispositif de guidage dynamique postérieur, comme proposé par exemple dans WO-A-03/094699. A cet effet, ce dispositif comporte, d'une part, des vis d'ancrage osseux dans le côté postérieur de deux vertèbres adjacentes et, d'autre part, des éléments élastiques de liaison entre ces vis. Ces éléments élastiques sont destinés en principe à soulager le disque intervertébral et à corriger d'éventuelles surpressions au niveau des surfaces articulaires entre ce disque et les vertèbres. Vis-à-vis d'un traitement par arthrodèse, ce genre de dispositif offre plus de confort au patient car il permet de conserver la mobilité du rachis. Cependant, en pratique, son utilisation se révèle délicate : le dimensionnement de l'élasticité des éléments de liaison est difficile puisqu'il doit être adapté à chaque patient selon sa pathologie et sa morphologie. De plus, à la longue, ces éléments risquent de voir leur comportement élastique évoluer. Le mauvais contrôle des paramètres relatifs à l'élasticité de ces éléments ne permet pas de garantir le respect de la cinématique recherchée pour le rachis, ce qui peut conduire à un guidage médiocre, voire à une instabilité de la distance intervertébrale et à une aggravation des lésions que l'on cherchait à traiter.

Le but de la présente invention est de proposer un dispositif de guidage dynamique postérieur du rachis, reproduisant plus fidèlement le mouvement anatomique des vertèbres, en guidant ces dernières de manière efficace, fiable et stable dans le temps.

A cet effet, l'invention a pour objet un dispositif de guidage dynamique postérieur du rachis, comportant deux ensembles vertébraux respectivement adaptés pour être fixés au côté postérieur de deux vertèbres adjacentes du rachis,
caractérisé en ce que les deux ensembles vertébraux définissent respectivement des surfaces d'articulation complémentaires sensiblement sphériques, qui, lorsque les deux ensembles vertébraux sont fixés aux vertèbres, s'étendent globalement du côté postérieur des vertèbres et glissent l'une contre l'autre, en étant centrées en un même point géométrique situé dans l'espace intervertébral séparant les deux vertèbres,
et en ce qu'il comporte des moyens mécaniques de limitation cinématique, adaptés pour limiter l'amplitude des glissements relatifs des surfaces d'articulation.

L'idée à la base de l'invention est de guider les mouvements relatifs des deux vertèbres respectivement munies des ensembles vertébraux, non pas par des éléments élastiques reliant ces ensembles, mais par la coopération directe de ces ensembles, au niveau de leur(s) surface(s) d'articulation sphérique(s) concentrique(s), agencée(s) du côté postérieur des vertèbres. De cette façon, la cinématique imposée aux vertèbres par la coopération de ces surfaces d'articulation est prédéterminée de manière précise, fiable et stable dans le temps. Cette cinématique s'apparente à celle d'une pseudo-rotule centrée dans l'espace intervertébral, de préférence dans la région centrale de cet espace, ce qui conduit les vertèbres à présenter un comportement articulaire dynamique proche de leur comportement anatomique normal, tant en flexion-extension qu'en torsion et en fléchissement latéral. En outre, la coopération de ces surfaces d'articulation soulage le disque intercalé entre les deux vertèbres : les dimensions, notamment la dimension verticale, de l'espace intervertébral sont en effet conservées, dans le sens où cet espace n'est pas réduit car le dispositif selon l'invention supporte l'essentiel, voire la totalité des contraintes liées aux mouvements du rachis. Autrement dit, grâce à l'action dynamique du dispositif, le disque intervertébral n'est pas mis en compression et conserve ainsi sa mobilité anatomique normale.

L'invention intègre également un contrôle de l'amplitude des mouvements relatifs des vertèbres ainsi guidées par les ensembles vertébraux, grâce aux moyens de limitation cinématique. Ces moyens sont efficaces et précis, en raison de leur nature mécanique. De plus, ils ont un double effet pratique : vis-à-vis des vertèbres, ils empêchent ces dernières de sortir d'un champ de liberté de mouvements préfixé, qui est avantageusement adapté à la morphologie du patient et/ou à la pathologie traitée, et, vis-à-vis des ensembles vertébraux du dispositif, ils peuvent maintenir les surfaces d'articulation au contact l'une de l'autre au niveau de parties substantielles de ces surfaces, à travers lesquelles les contraintes mécaniques sont efficacement transmises entre les ensembles vertébraux.

En outre, l'implantation du dispositif selon l'invention se révèle particulièrement facile : la mobilité interne au dispositif réside essentiellement, voire exclusivement au niveau des surfaces d'articulation définies par les ensembles vertébraux dont les positions d'ancrage dans le côté postérieur des vertèbres à traiter sont choisies et fixées par le chirurgien. Par ailleurs, comme ces surfaces d'articulation s'étendent postérieurement au rachis, les gestes chirurgicaux d'implantation sont concentrés à l'arrière du rachis.

D'autres caractéristiques avantageuses du dispositif conforme à l'invention, prises isolément ou suivant toutes les combinaisons techniquement possibles, sont énoncées aux revendications dépendantes 2 à10

L'invention a également pour objet un ensemble de guidage dynamique postérieur du rachis, tel que défini à la revendication 11. Cet ensemble de guidage selon l'invention permet de traiter trois ou davantage de vertèbres adjacentes.

L'invention a en outre pour objet un système de traitement du rachis, caractérisé en ce qu'il comprend, d'une part, un dispositif de guidage dynamique postérieur du rachis, tel que défini ci-dessus, et, d'autre part, un moyen de blocage temporaire de ce dispositif, adapté pour être relié de façon amovible aux ensembles vertébraux et pour bloquer alors les glissements relatifs des surfaces d'articulation.

Ce moyen de blocage facilite l'implantation du dispositif de guidage, en neutralisant temporairement la mobilité interne de ce dispositif, en particulier pendant la fixation des ensembles vertébraux aux vertèbres. Le chirurgien peut alors ajuster avec précision la position d'ancrage des ensembles vertébraux, notamment de manière à positionner le centre géométrique des surfaces d'articulation de manière adéquate, de préférence dans la région centrale de l'espace intervertébral. Une fois que ces ensembles vertébraux sont fixés aux vertèbres, le moyen de blocage est dégagé par le chirurgien et l'intervention d'implantation du dispositif peut prendre fin.

Une caractéristique avantageuse de ce système est énoncée à la revendication 13.

L'invention sera mieux comprise à la lecture de la description qui va suivre, donnée uniquement à titre d'exemple et faite en se référant aux dessins sur lesquels :
- les figures 1 et 2 sont des vues en élévation, respectivement latérale et postérieure, d'un dispositif conforme à l'invention, représenté de manière schématique dans une configuration d'implantation à un rachis;
- la figure 3 est une coupe selon la ligne III-III de la figure 2 ;
- la figure 4 est une vue en perspective éclatée d'un système de traitement conforme à l'invention, comportant le dispositif des figures 1 à 3 ;
- la figure 5 est une vue en perspective du système de la figure 4, dans une configuration d'utilisation de ce système ;
- la figure 6 est une vue analogue à la figure 3, illustrant un aménagement optionnel du dispositif ;
- la figure 7 est une vue en perspective d'une variante du dispositif conforme à l'invention ;
- la figure 8 est une vue en perspective d'un second mode de réalisation d'un dispositif conforme à l'invention ;
- les figures 9 et 10 sont des vues analogues aux figures 1 et 2, illustrant le second mode de réalisation du dispositif ;
- la figure 11 est une coupe selon le plan XI-XI de la figure 9;
- la figure 12 est une vue analogue à la figure 10, illustrant une variante du second mode de réalisation, conforme à l'invention ;
- la figure 13 est une vue en perspective d'un ensemble de guidage conforme à l'invention ; et
- la figure 14 et une vue analogue à la figure 1, illustrant l'ensemble de la figure 13 dans une configuration d'implantation à un rachis.

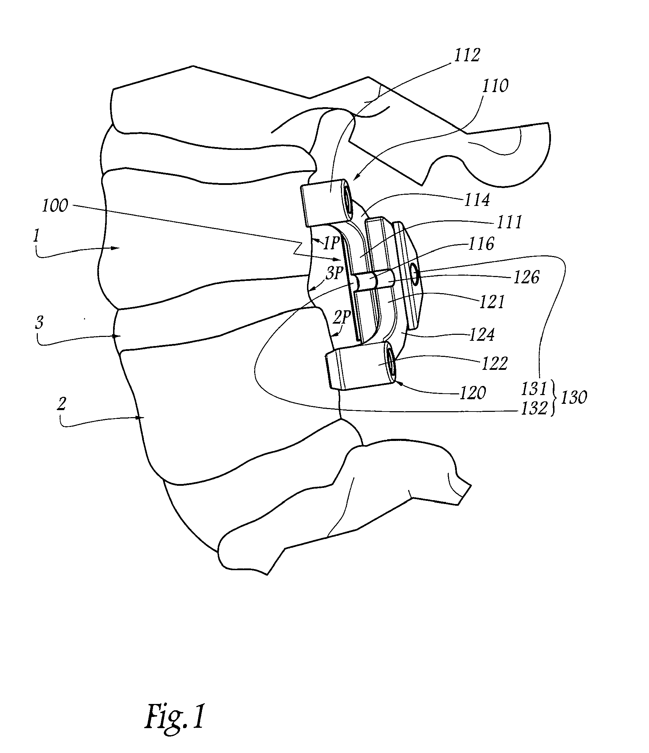

Sur les figures 1 à 3 sont représentées deux vertèbres adjacentes 1 et 2 d'un rachis lombaire d'un patient humain. Ces vertèbres sont séparées l'une de l'autre par un disque intervertébral 3. Par commodité, la suite de la description est orientée par rapport à ces vertèbres dans leur position anatomique, c'est-à-dire que les termes « postérieur » ou « arrière », « antérieur » ou « avant », « droit », « gauche », « supérieur », « inférieur », etc. s'entendent par rapport au rachis du patient se tenant debout.

Sur les figures 1 à 5 est représenté un dispositif 100 de guidage des vertèbres 1 et 2, implanté sur le côté postérieur des vertèbres et apte à fournir une liaison mécanique articulaire postérieure entre ces vertèbres, tout en maintenant l'espace intervertébral qui sépare verticalement ces vertèbres et qui est occupé par le disque 3. Le dispositif 100 comporte un ensemble vertébral supérieur 110 implanté sur la vertèbre 1 et un ensemble vertébral inférieur 120 implanté sur la vertèbre 2.

Chaque ensemble vertébral 110, 120 comporte une coupelle 111, 121 rigide, notamment en métal ou en céramique. Les deux coupelles 111 et 121 s'étendent globalement du côté postérieur des vertèbres 1 et 2, en étant imbriquées l'une dans l'autre, la coupelle 111 étant disposée à l'avant et à l'intérieur de la coupelle 121. Les deux coupelles présentent ainsi leur concavité respective tournée vers le côté postérieur des vertèbres.

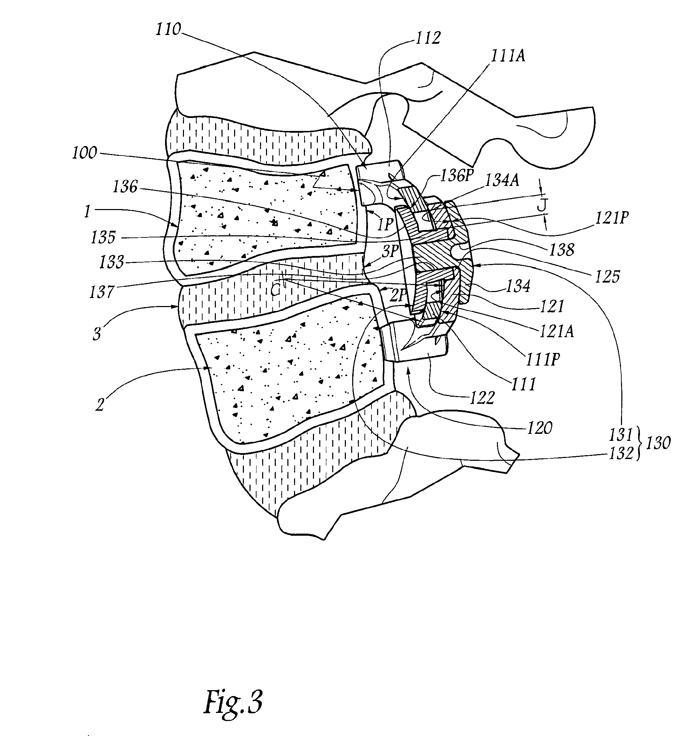

La coupelle 111 délimite, d'une part, une surface antérieure 111A concave, s'étendant, de haut en bas, en regard direct successivement des faces postérieures 1P, 3P et 2P, respectivement, de la vertèbre 1, du disque 3 et de la vertèbre 2, et, d'autre part, une surface postérieure 111P convexe. La coupelle 121 délimite, quant à elle, d'une part, une surface antérieure 121A concave et complémentaire de la surface 111P, et, d'autre part, une surface postérieure 121P convexe. Comme bien visible à la figure 3, les surfaces 111A, 111P, 121A et 121P sont des surfaces sphériques correspondant respectivement à des portions de sphère en forme de calottes, toutes centrées en un même point géométrique C situé dans l'espace intervertébral occupé par le disque 3.

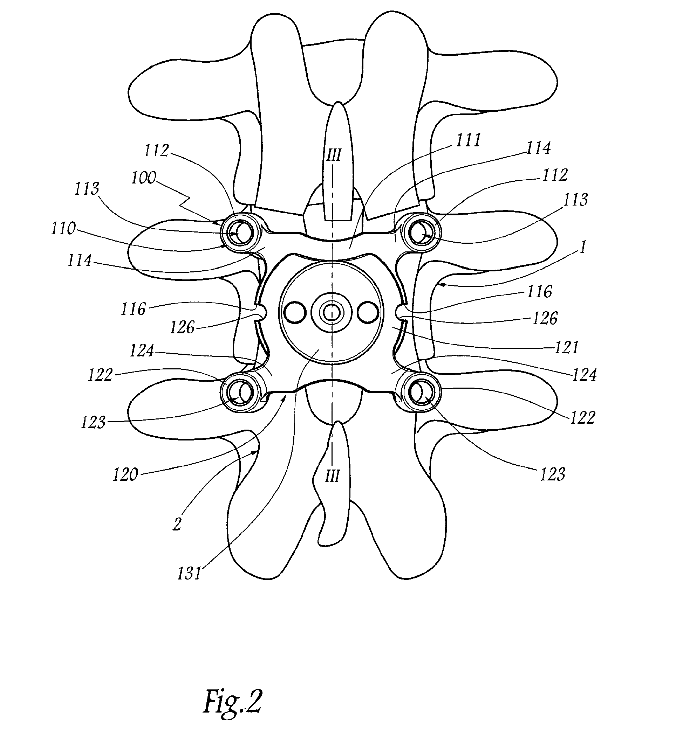

Vues en élévation postérieure, comme à la figure 2, les coupelles 111 et 121 s'étendent globalement sur l'essentiel de la dimension médio-latérale des vertèbres 1 et 2. Ainsi, les surfaces sphériques 111A et 111P, 121A et 121P de ces coupelles s'étendent de part et d'autre du plan sagittal et de leur vertèbre associée 1, 2, ce plan correspond à la ligne III-III sur la figure 2.

Chaque coupelle 111, 121 est munie de deux plots de fixation osseuse 112, 122, sous forme de tubes délimitant intérieurement des orifices traversants 113, 123 débouchant sur la face postérieure 1P, 2P de leur vertèbre associée 1, 2. Chaque orifice 113, 123 est prévu pour recevoir une vis, non représentée, d'ancrage dans la matière osseuse constituant la vertèbre 1, 2 pour immobiliser l'ensemble 110, 120 à sa vertèbre associée. Les deux plots 112 sont respectivement situés en des zones périphériques supérieures de la coupelle 111, tandis que les deux plots 122 sont respectivement situés dans des zones périphériques inférieures de la coupelle 121. Avantageusement, les deux plots 112, respectivement 122, sont ainsi alignés suivant une direction médiolatérale, de part et d'autre du plan sagittal de la vertèbre 1, respectivement 2, de manière que leurs orifices 113, respectivement 123, débouchent sur les pédicules de la vertèbre, qui sont des régions postérieures de la vertèbre où la matière osseuse est de bonne qualité pour s'y ancrer.

A cet effet, chaque plot 112, 122 est respectivement solidaire de sa coupelle 111, 121 par une patte 114, 124 reliant rigidement une portion de la périphérie extérieure de la coupelle à la face extérieure du tube formant le plot. Chaque patte 114, 124 s'étend dans le prolongement sphérique de la coupelle 111, 121, c'est-à-dire que les faces antérieure et postérieure de chaque patte appartiennent respectivement aux enveloppes sphériques définies par la surface antérieure 111A, 121A et par la surface postérieure 111P, 121P de la coupelle 111, 121. Les tubes formant les plots 112 et 122 sont transversaux à leur patte correspondante 114, 124. Les pattes 114 et 124 correspondent ainsi chacune à des prolongements périphériques continus de matière de la coupelle 111, 121, de même courbure que la coupelle, ce qui facilite la fabrication de ces pattes, notamment en prévoyant qu'elles sont moulées et/ou usinées de façon monobloc avec la coupelle.

Ainsi, chaque ensemble 110, 120 définit avantageusement un plan vertical de symétrie, qui passe par le point C et qui est sensiblement confondu avec le plan sagittal de la vertèbre 1, 2.

Les plots 122 présentent une dimension antéropostérieure supérieure à celle des plots 121, de sorte que, lorsque le dispositif 100 est en configuration d'implantation, c'est à dire lorsque les ensembles vertébraux 110 et 120 sont fixés aux vertèbres 1 et 2 par insertion et serrage de vis d'ancrage osseux dans les plots 112 et 122, la coupelle 121 recouvre la coupelle 111, avec les surfaces 111P et 121A appuyées directement l'une contre l'autre.

Ainsi, lorsque le dispositif 100 est en configuration d'implantation, les surfaces 111P et 121A coopèrent l'une avec l'autre du côté postérieur du rachis, par complémentarité de formes, en pouvant glisser l'une contre l'autre de manière centrée sur le point C. Ces surfaces 111P et 121A constituent donc des surfaces d'articulation entre les ensembles vertébraux 110 et 120, les glissements entre ces surfaces correspondant à des mouvements de rotation centrés en C, de sorte que la liaison cinématique entre ces deux ensembles s'apparente à une liaison rotule de centre C. En effet, les glissements relatifs des deux surfaces 111P et 121A sont alors possibles dans toutes les directions de l'espace, tant qu'est maintenu le contact entre ces surfaces. Ainsi, dans la mesure où le centre d'articulation C est situé dans l'espace interdiscal, en particulier dans la région centrale de cet espace, la cinématique imposée aux ensembles vertébraux 110 et 120 par la coopération des surfaces 111P et 121A conduit les vertèbres 1 et 2 à présenter un comportement dynamique relatif identique ou, tout au moins, proche de leur comportement anatomique normal, tant en flexion-extension, qu'en torsion et en fléchissement gauche/droite. En particulier, la coopération sphérique postérieure des coupelles 111 et 121 est prévue pour maintenir l'écartement vertical des vertèbres 1 et 2, évitant la mise en contrainte du disque 3 qui conserve ainsi sa mobilité naturelle. La forme en calotte des surfaces d'articulation 111A et 121P garantit une bonne homogénéité du guidage dynamique des ensembles 110 et 120, donc des vertèbres 1 et 2, dans toutes les directions de sollicitation du rachis.

Pour limiter l'amplitude des glissements entre les surfaces d'articulation 111P et 121A, le dispositif 100 intègre des moyens de limitation cinématique correspondants 130. Dans l'exemple considéré ici, ces moyens 130 comportent deux pièces distinctes 131 et 132, rapportées de part et d'autre des coupelles 111 et 121 suivant une direction antéropostérieure. La pièce postérieure 131 comporte un téton cylindrique 133 muni, à son extrémité longitudinale arrière, d'un bord périphérique saillant 134. La surface antérieure 134A du bord 134 est complémentaire de la surface 121P pour appuyer ces surfaces directement l'une contre l'autre à l'état assemblé des pièces 131 et 132. A cet effet, la coupelle 121 est traversée de part en part, suivant une direction antéropostérieure, par un orifice traversant 125, de section transversale ajustée à celle du téton 133, comme bien visible à la figure 3.

La pièce antérieure 132 comporte un pion cylindrique 135 muni, à son extrémité longitudinale avant, d'un bord périphérique saillant 136. La surface postérieure 136P du bord 136 est complémentaire de la surface 111A pour appuyer ces surfaces directement l'une contre l'autre. Le pion 135 délimite intérieurement un trou longitudinal 137 de réception et d'immobilisation de la partie avant du téton 133. En particulier, le trou 137 et le téton 133 peuvent être prévus respectivement taraudé et fileté de manière complémentaire, de telle sorte que la pièce 131 se trouve solidarisée rigidement à la pièce 132 par vissage. Pour faciliter ce vissage, la surface postérieure 134P du bord 134 est avantageusement munie d'une empreinte d'entraînement de la pièce 131 en rotation sur elle-même, constituée ici de deux creux diamétralement opposés 139. De manière plus générale, la coopération du téton 133 et du trou 137 est prévue pour assembler fixement les pièces 131 et 132 l'une à l'autre.

Le pion 135 présente, d'une part, une longueur antéropostérieure suffisante pour à la fois traverser de part en part la coupelle 111, via un orifice traversant 115 de cette coupelle, et atteindre la surface 121A, et, d'autre part, une section transversale non circulaire, mais de forme oblongue. En prévoyant que le débouché antérieur de l'orifice 125 présente une section transversale oblongue ajustée sur celle du pion 135, la mise en place de l'extrémité postérieure de ce pion dans ce débouché lors de l'assemblage des pièces 131 et 132 immobilise en rotation la pièce 132 et la coupelle 121 l'une par rapport à l'autre. Comme la coupelle 121 est alors également bloquée par la coupelle 111 vers l'avant et par le bord 134 vers l'arrière, la coupelle 121 se trouve ainsi liée de manière fixe aux pièces 131 et 132.

A la différence de l'orifice 125, l'orifice 115 présente une section transversale qui n'est pas ajustée sur celle du pion 135. Au contraire, la section de cet orifice présente des dimensions plus grandes que celles du profil extérieur du pion 135, et ce dans toutes les directions du plan transversal à ce pion. Dans l'exemple de réalisation considéré ici, l'orifice 115 présente une section transversale de forme oblongue, sensiblement homothétique de celle du profil extérieur de la section transversale du pion 135, avec les axes principaux respectifs de ces deux formes oblongues qui s'étendent globalement suivant une direction verticale, tandis que leur petit axe respectif s'étend dans une direction sensiblement médiolatérale. De la sorte, lorsque les pièces 131 et 132 sont assemblées l'une à l'autre, le pion 135 s'étend en travers de l'orifice 115 et des jeux non négligeables, à la fois verticaux et médiolatéraux, existent entre le pion 135 et la paroi de la coupelle 111 délimitant l'orifice 115, comme visible pour les jeux verticaux notés J à la figure 3. On comprend que, lors des glissements relatifs des surfaces d'articulation 111P et 121A, le pion 115 change de position à l'intérieur de l'orifice 115, jusqu'à, le cas échéant, venir buter contre une portion périphérique de la paroi délimitant cet orifice, ce qui limite donc l'amplitude des glissements entre les coupelles 111 et 121.

Avantageusement, le bord 136 délimite une surface postérieure 136P complémentaire de la surface 111A, contre laquelle la surface 136P est directement appuyée lorsque les pièces 131 et 132 sont assemblées l'une à l'autre. De la sorte, les bords 134 et 136 permettent de maintenir la configuration d'assemblage du dispositif 100, c'est-à-dire permettent de maintenir entre eux les coupelles 111 et 121 appuyées l'une contre l'autre, dans la mesure où, lorsque les surfaces 111P et 121A glissent l'une contre l'autre, les surfaces 111A et 136P glissent l'une contre l'autre de manière correspondante.

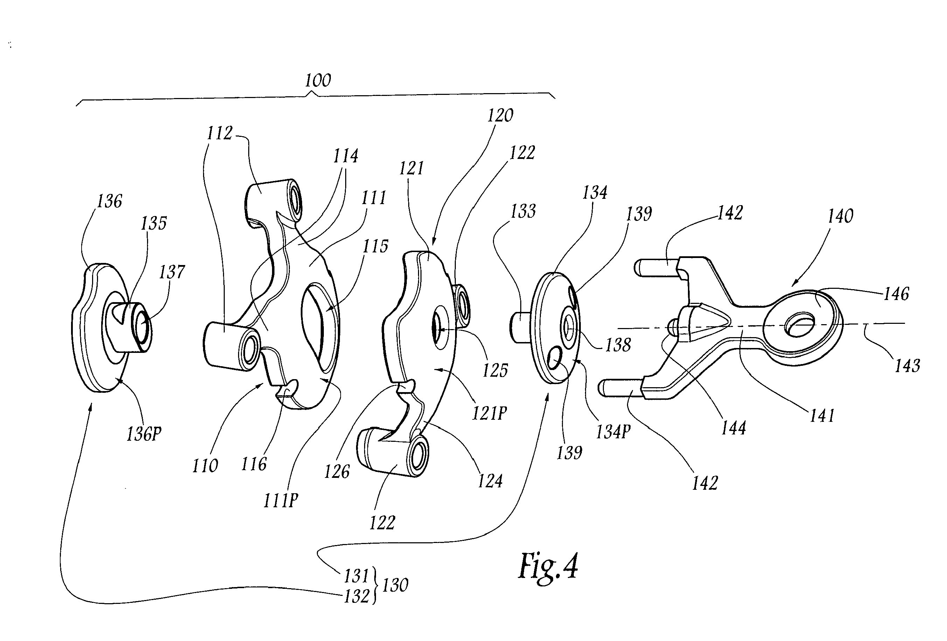

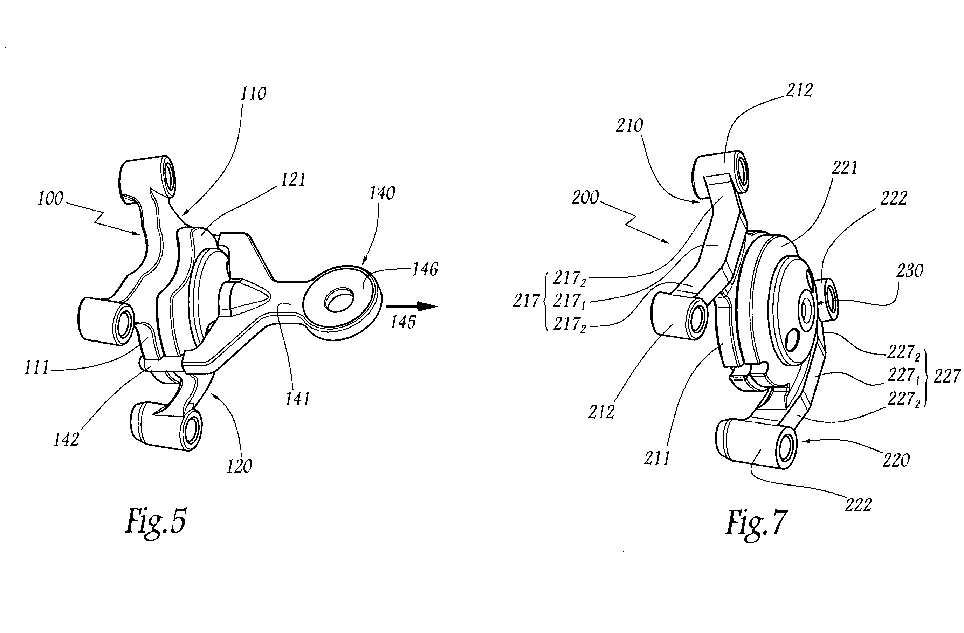

Le dispositif 100 est avantageusement associé à une fourche 140 représentée sur les figures 4 et 5. Cette fourche comporte un corps principal allongé 141 muni, à l'une de ses extrémités longitudinales, de deux broches parallèles 142 s'étendant parallèlement et de manière symétrique l'un de l'autre à un axe longitudinal central 143 du corps 141. Chaque broche 142 est adaptée pour être reçue en longueur dans deux encoches 116 et 126 délimitées respectivement par les coupelles 111 et 121. Les deux encoches 116 de la coupelle 111 sont diamétralement opposées suivant la périphérie extérieure de cette coupelle, en étant alignées suivant une direction médiolatérale. De même, les deux encoches 126 de la coupelle 121 sont diamétralement opposées suivant la périphérie extérieure de cette coupelle, en étant également alignées suivant une direction médiolatérale. De cette façon, les encoches 116 et 126 débouchent deux à deux l'une dans l'autre lorsque les régions centrales des coupelles 111 et 121 sont alignées suivant une direction antéropostérieure, c'est-à-dire lorsque les surfaces d'articulation 111P et 121A sont appuyées l'une contre l'autre de manière centrée, comme sur les figures 1 à 3 et 5. Dans cette position relative des coupelles 111 et 121, les broches 142 peuvent être introduites, d'arrière en avant, dans successivement les encoches 126 et 116 et, par complémentarité de formes entre ces broches et ces encoches, la fourche 140 bloque les coupelles l'une par rapport à l'autre, comme représenté à la figure 5.

Avantageusement, l'extrémité antérieure du corps 141 est munie, en plus des deux broches 142, d'un téton cylindrique 144 centré sur l'axe 143 et adapté pour être reçu de manière complémentaire dans un évidement associé 138 de la pièce 131, débouchant sur la surface 134P. La mise en place du téton 144 dans l'évidement 138 parfait la mise en place de la fourche 140, dans le sens où le téton et l'évidement présentent avantageusement des aménagements provoquant le clipsage du téton dans l'évidement, de sorte que le dégagement de la fourche 140 nécessite alors un effort de déclipsage correspondant, ce qui limite les risques de désengagement accidentel de la fourche.

Pour implanter le dispositif 100 sur les vertèbres 1 et 2, un chirurgien dégage, par une voie d'abord postérieure, les parties molles du patient situées du côté postérieur des vertèbres. Il résèque ensuite les apophyses épineuses des vertèbres 1 et 2, ainsi que, si nécessaire, la partie inférieure de l'apophyse de la vertèbre située juste au-dessus de la vertèbre 1 et la partie supérieure de l'apophyse de la vertèbre située juste au-dessous de la vertèbre 2, comme représenté sur les figures 1 à 3. En pratique, cette résection apophysaire doit être suffisante pour permettre d'amener le dispositif 100 de l'arrière vers l'avant, jusqu'au contact des faces postérieures 1 P et 2P des vertèbres 1 et 2, avec les plots 112 et 122 débouchant directement contre ces faces osseuses pour pouvoir y introduire et visser des vis d'ancrage, non représentées, par voie postérieure.

Le chirurgien dispose à cet effet du dispositif 100 dans un état assemblé, c'est-à-dire avec les ensembles 110 et 120 assemblés et maintenus l'un à l'autre par les pièces 131 et 132 solidaires l'une de l'autre. En outre, durant la mise en place et la fixation du dispositif 100 le long des vertèbres 1 et 2, la fourche 140 est utilisée de manière à bloquer les mouvements relatifs des coupelles 111 et 121, c'est-à-dire que le dispositif 100 est manipulé conjointement avec la fourche 140, dans leur configuration d'assemblage de la figure 5. De cette façon, le chirurgien fixe les ensembles vertébraux 110 et 120 aux vertèbres 1 et 2 alors que ces deux ensembles sont immobilisés, de manière centrée, l'un par rapport à l'autre. La précision d'implantation du dispositif 100 s'en trouve améliorée. En outre, comme la fourche 140 neutralise la mobilité interne du dispositif 100, les gestes chirurgicaux sont grandement facilités pour le chirurgien, qui, lors de la fixation du dispositif 100 aux vertèbres 1 et 2, n'a pas à se soucier du positionnement relatif des ensembles 110 et 120.

En pratique, si besoin, le dispositif 100 est implanté en étant mis en contrainte afin de soulager le disque 3, c'est-à-dire que les ensembles 110 et 120 sont fixés aux vertèbres 1 et 2 alors que ces dernières sont maintenues écartées l'une de l'autre de manière prédéterminée, par un ancillaire adéquat. La valeur de l'écartement imposé entre les vertèbres peut être prévue égale à celle d'un écartement anatomique normal ou être volontairement surdimensionnée par rapport à cet écartement normal, selon la pathologie traitée. Par exemple, pour traiter un écrasement vertébral affectant principalement les côtés postérieurs des vertèbres, un tel surdimensionnement peut s'avérer très bénéfique, notamment par le fait qu'il soulage efficacement la gêne et la douleur ressenties par le patient.

Une fois que le dispositif 100 est fixé aux vertèbres 1 et 2, la fourche 140 est dégagée, en déclipsant le téton 144 de l'évidement 138 et en retirant les fourches 142 des évidements 116 et 126. Pour ce faire, le chirurgien tire vers l'arrière le corps 141 suivant son axe 143, comme indiqué par la flèche 145 à la figure 5, en agissant avantageusement sur une palette de traction 146 prévue à l'extrémité du corps 141 opposée à celle munie des broches 142.

Le dispositif 100 se trouve alors dans sa configuration d'implantation des figures 1 à 3. Le chirurgien referme ensuite les parties molles postérieures aux vertèbres 1 et 2 et l'intervention chirurgicale prend fin. Après implantation, le dispositif conserve à demeure sa mobilité interne, liée aux glissements des surfaces d'articulation 111P et 121A l'une contre l'autre, dans la limite des débattements imposés par les moyens 130.

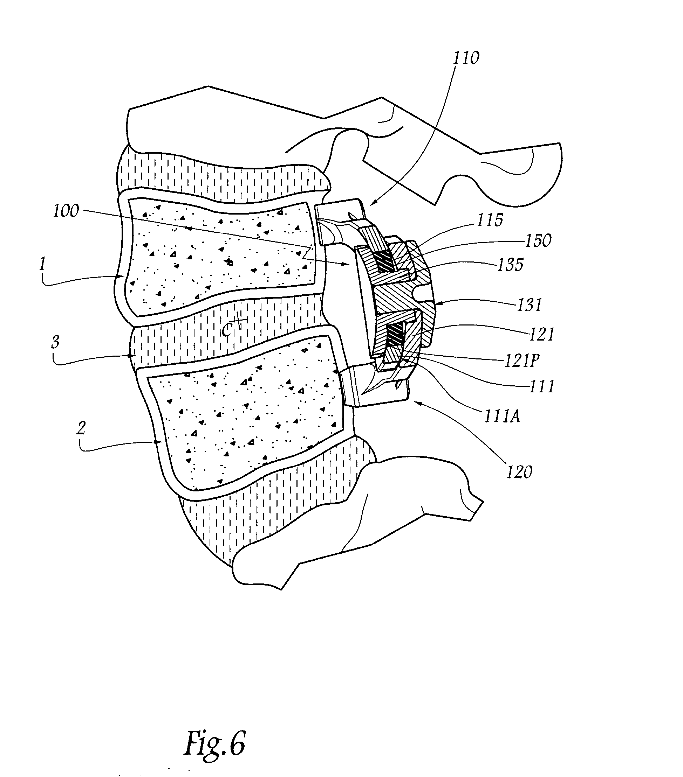

Sur la figure 6, le dispositif 100 des figures 1 à 5 est représenté avec un aménagement optionnel, consistant en une rondelle 150 réalisée en une matière souple et occupant le volume libre entre le pion 135 et la paroi du trou 115, lié aux jeux de débattement entre les coupelles 111 et 121. Cette rondelle 150 est ainsi interposée, de manière globalement radiale à l'axe longitudinal central du pion 135, entre ce pion et la paroi de l'orifice 115.

En service, lorsque les surfaces 111 P et 121A glissent l'une contre l'autre, la rondelle 150 amortit les glissements relatifs entre les coupelles 111 et 121, évitant des mises en butée trop brutales du pion 135 contre la paroi de l'orifice 115, ce qui limite la gène correspondante pour le patient.

Sur la figure 7 est représentée une variante 200 du dispositif 100. Le dispositif 200 comporte deux ensembles vertébraux supérieur 210 et inférieur 220 dont les coupelles respectives 211 et 221 sont identiques aux coupelles 111 et 121 des ensembles 110 et 120 du dispositif 100. Le dispositif 200 comporte également des moyens mécaniques de limitation cinématique 230, identiques aux moyens 130 du dispositif 100. Le dispositif 200 se distingue du dispositif 100 par la forme de réalisation du lien rigide entre chacune de ses coupelles 211 et 221 et leur paire de plots de fixation osseuse 212 et 222. En effet, en remplacement des pattes 114 et 124 prévues dans le dispositif 100, le dispositif 200 comporte, pour chaque ensemble vertébral 210, 220, une poutre 217, 227. Chaque poutre 217, 227 comporte une partie courante 217₁, 227₁ s'étendant en longueur suivant une direction médiolatérale et prolongée à chacune de ses extrémités longitudinales par une partie terminale 217₂, 227₂ coudée vers l'avant. A leur extrémité opposée, les parties terminales 217₂, 227₂ sont liées rigidement aux plots 212, 222, tandis que la coupelle 111, 121 est liée rigidement, sur une portion correspondante de sa périphérie extérieure, à la partie courante 217₁,227₁.

Les poutres 217 et 227 sont plus encombrantes que les pattes 114 et 124, mais correspondent à une structure mécaniquement plus robuste, la forme en poutre renforçant l'encaissement des contraintes dynamiques transmises entre la coupelle 211, 221 et la vertèbre correspondante 1, 2.

L'implantation du dispositif 200 est identique à celle du dispositif 100, l'utilisation de la fourche 140 étant en particulier possible.

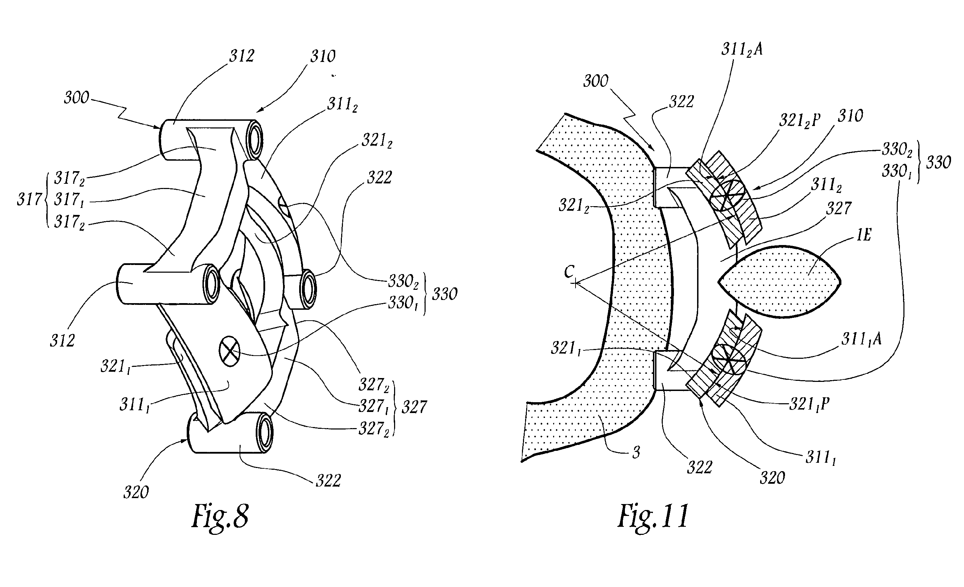

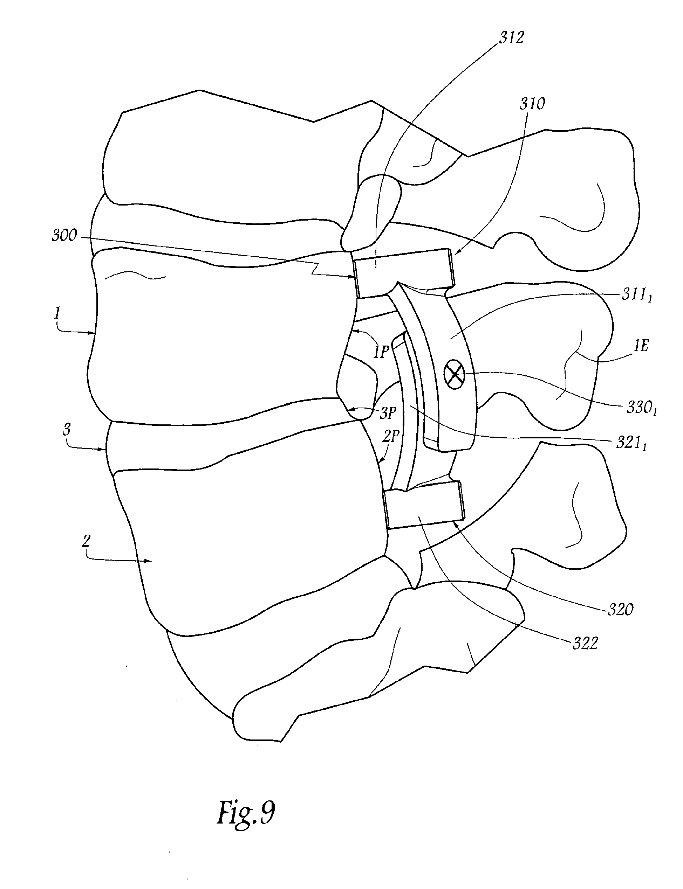

Sur les figures 8 à 11 est représenté un second mode de réalisation d'un dispositif 300 de guidage des vertèbres 1 et 2, dont le comportement dynamique et les effets sur ces vertèbres sont sensiblement identiques à ceux du dispositif 100 ou 200.

Le dispositif 300 comporte, d'une part, un ensemble vertébral supérieur 310 fixé à la face postérieure 1 P de la vertèbre 1 par deux vis d'ancrage, non représentées, reçues dans deux plots de fixation 312 reliés rigidement l'un à l'autre par une poutre 317, ces plots 312 et la poutre 317 présentant une structure identique aux plots 212 et à la poutre 217 du dispositif 200, et, d'autre part, un ensemble vertébral inférieur 320 fixé à la face 2P de la vertèbre 2 par deux vis d'ancrage, non représentées, reçues dans deux plots de fixation 322 reliés rigidement l'un à l'autre par une poutre 327, avec une structure identique à celle des plots 222 et de la poutre 227 de l'ensemble 220 du dispositif 200.

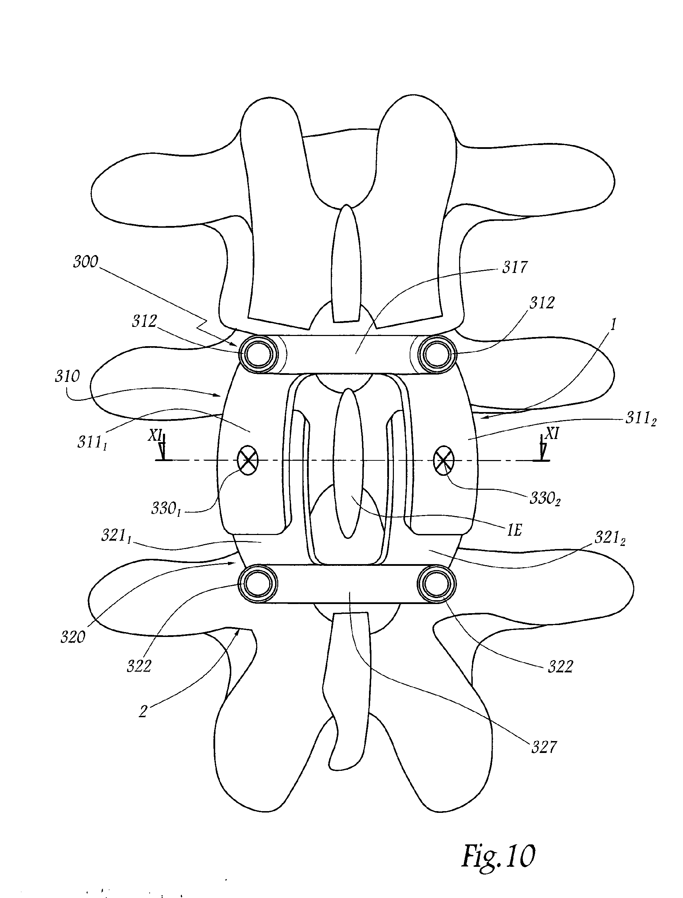

Le dispositif 300 se distingue du dispositif 200 par l'agencement de la coopération en glissement entre ses ensembles 310 et 320. En effet, plutôt qu'une unique coupelle en forme de calot, comme les coupelles 211 et 221 du dispositif 200, chaque ensemble 310, 320 comporte deux coupelles 311₁ et 311₂, 321₁ et 321₂ en forme de quartiers de sphère. Ces deux coupelles 311₁ et 311₂, 321₁ et 321₂ sont distantes l'une de l'autre suivant une direction antéropostérieure, de manière à délimiter entre elles un espace libre s'étendant de part et d'autre du plan sagittal de leur vertèbre associée 1, 2. Comme bien visible aux figures 10 et 11, lorsque le dispositif 300 est implanté, une partie substantielle de l'apophyse 1 E de la vertèbre 1 s'étend vers l'arrière en travers de cet espace libre, de sorte que l'implantation du dispositif 300 nécessite une moindre résection apophysaire que l'implantation des dispositifs 100 et 200, étant remarqué que des résections localisées des apophyses restent nécessaires en raison de la partie courante 317₁, 327₁ des poutres 317 et 327.

Les coupelles 311₁ et 311₂, 321₁ et 321₂ sont respectivement solidarisées de manière rigide aux deux parties terminales 317₂, 327₂ de leur poutre 317, 327.

Pour permettre l'articulation relative des ensembles 310 et 320 de manière analogue à celle des ensembles 210 et 220 du dispositif 200, chaque coupelle 311₁, 311₂, 321₁, 321₂ présente une concavité tournée vers le côté postérieur des vertèbres 1 et 2, de manière que la surface sphérique antérieure 311₁A, 311₂A de chacune des coupelles 311₁ et 311₂ recouvre de manière complémentaire la surface sphérique postérieure 321₁P, 321₂P d'une des deux coupelles 321₁ et 321₂, toutes ces surfaces sphériques 311₁A, 311₂A, 321₁P et 321₂P étant centrées en un même point géométrique C situé dans l'espace intervertébral séparant les vertèbres 1 et 2, comme bien visible à la figure 11. Le point C correspond au centre de la sphère géométrique dont les surfaces 311₁A, 311₂A, 321₁P et 321₂P sont des portions en forme de quartiers surfaciques. De la sorte, les deux surfaces 311₁A, 311₂A, considérées conjointement, forment, pour moitié chacune, une surface d'articulation fonctionnellement analogue à la surface antérieure 121A de l'ensemble 120, vis-à-vis de la surface d'articulation formée, pour moitié chacune, des surfaces 321₁P, 321₂P et fonctionnellement analogue à la surface postérieure 111 P pour l'ensemble 110.

Par ailleurs, le dispositif 300 comporte des moyens mécaniques de limitation cinématique 330, fonctionnellement analogues aux moyens 130. Ces moyens 330 sont constitués de deux parties respectivement associées aux paires de coupelles 311₁ et 321₁, et 311₂ et 321₂, pour respectivement limiter l'amplitude des glissements des surfaces 311₁A et 321₁P, et 311₂A et 321₂P. Par commodité, la structure des parties 330₁ et 330₂ n'est pas représentée en détail sur les figures 8 à 11, chacune de ces parties 330₁ et 330₂ étant indiquée uniquement de manière schématique par une zone marquée d'une croix sur ces figures. Suivant un mode de réalisation pratique, chaque partie 330₁, 330₂ présente la même structure que les moyens 130, en incluant notamment des pièces analogues aux pièces 131 et 132.

On notera que, en raison de la liaison rigide constituée par les poutres 317 et 327, une variante non représentée consiste à ne prévoir qu'une seule des parties 330₁ et 330₂. Toutefois, la présence des deux parties 330₁ et 330₂ permet de limiter l'amplitude des mouvements relatifs entre les ensembles 310 et 320 de manière homogène dans toutes les directions de sollicitation envisageables, notamment lorsque les vertèbres 1 et 2 sont sollicitées en fléchissement gauche/droite.

L'implantation du dispositif 300 est analogue à celle du dispositif 100, notamment en utilisant une ou deux fourches, fonctionnellement analogues à la fourche 140 et à même de bloquer respectivement les coupelles 311₁ et 321₁, et 311₂ et 321₂ l'une par rapport à l'autre.

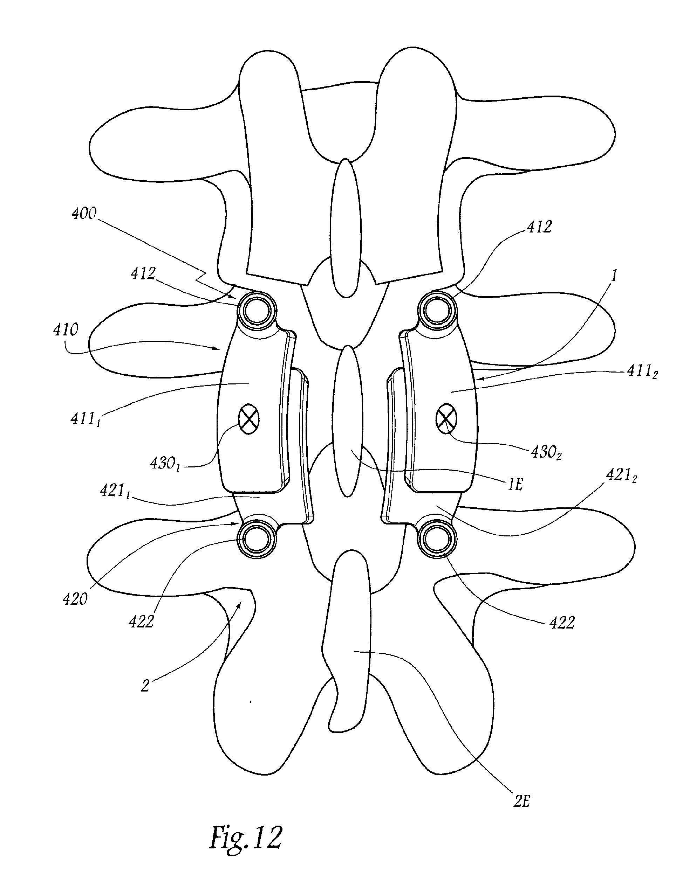

Sur la figure 12 est représentée une variante 400 du dispositif 300, qui ne se distingue du dispositif 300 que par l'absence de la partie courante 317₁, 327₁ des poutres 317 et 327. Ainsi, le dispositif 400 comporte des ensembles vertébraux supérieur 410 et inférieur 420, dont les deux coupelles respectives 321₁ et 321₂, 421₁ et 421₂ sont mécaniquement indépendantes l'une de l'autre, dans le sens où elles ne sont pas reliées l'une à l'autre en service par des constituants du dispositif 400. L'implantation du dispositif 400 ne nécessite ainsi aucune résection des apophyses 1 E et 2E des vertèbres 1 et 2, ainsi que de celles de leur vertèbre immédiatement adjacente.

Le dispositif 400 comporte par ailleurs des moyens mécaniques 430 permettant de limiter l'amplitude des glissements relatifs de chaque paire de coupelles associées 411₁ et 421₁, et 411₂ et 421₂, respectivement sous la forme de deux parties 430₁ et 430₂ analogues aux parties 330₁ et 330₂ des moyens 330 du dispositif 300.

L'implantation du dispositif 400 est sensiblement analogue à celle du dispositif 300, de préférence avec l'utilisation de deux fourches de blocage des coupelles 411₁ et 421₁, et 411₂ et 421₂, l'une par rapport à l'autre, du type de la fourche 140.

Divers aménagements et variantes aux dispositifs 100, 200, 300 et 400 décrits ci-dessus sont par ailleurs envisageables. A titre d'exemples :
- si on considère, par exemple, le dispositif 100, la structure de recouvrement des coupelles 111 et 121 peut être inversée de sorte que, en variante non représentée, la coupelle de l'ensemble vertébral supérieur 110 recouvre postérieurement la coupelle 121 de l'ensemble vertébral inférieur 120 ; une telle inversion de la structure de recouvrement des deux ensembles vertébraux est d'ailleurs illustrée pour les dispositifs 300 et 400 considérés ci-dessus ; et/ou
- d'autres formes de réalisation que celle considérée aux figures peuvent être envisagées pour les moyens de limitation cinématique 130, 230, 330 et 430 ; en particulier, si on renonce à ce que ces moyens de limitation soient répartis de part et d'autre, suivant une direction antéropostérieure, des coupelles articulées l'une sur l'autre en glissement, un pion, fonctionnellement analogue au pion 135, peut être directement venu de matière avec l'une des deux coupelles, pour être reçu, avec des jeux de débattement, dans un orifice, fonctionnellement analogue à l'orifice 115, ménagé par l'autre coupelle, en prévoyant de rapporter autour de l'extrémité libre de ce pion une rondelle et un écrou d'assemblage.

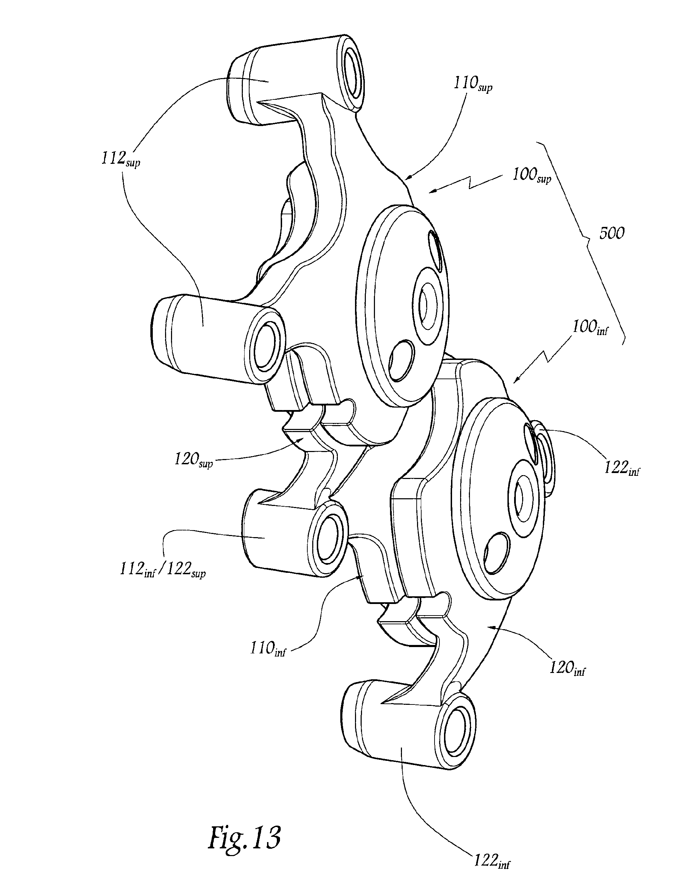

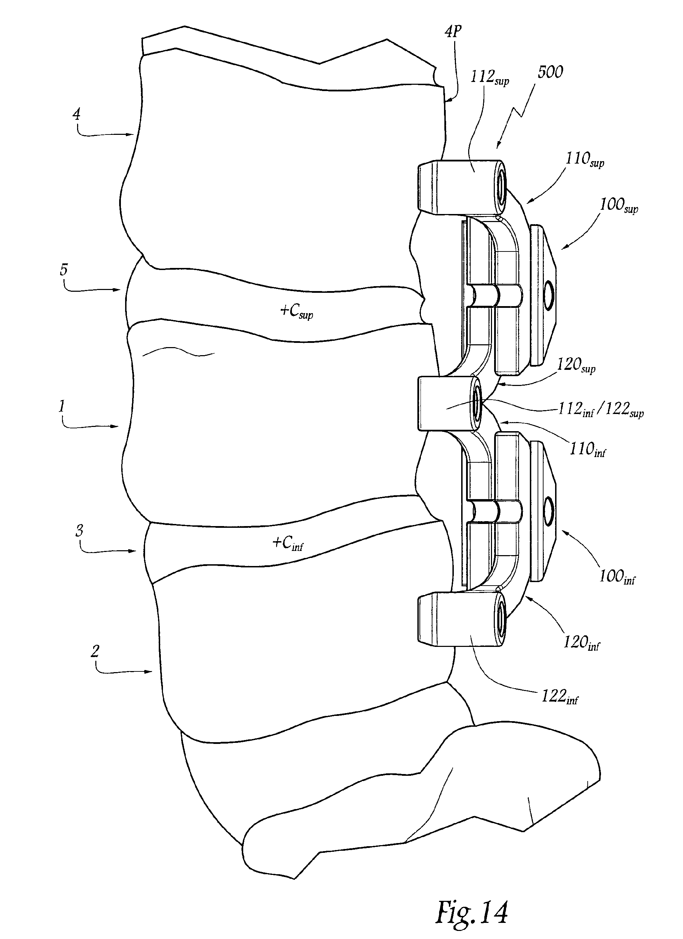

Sur les figures 13 et 14 est représenté un ensemble 500 de guidage de trois vertèbres adjacentes. Cet ensemble 500 comporte à cet effet deux dispositifs analogues au dispositif 100 des figures 1 à 5, à savoir un dispositif inférieur 100_{inf} et un dispositif supérieur 100ₛᵤₚ. Le dispositif 100_{inf} est identique au dispositif 100 illustré sur les figures 1 à 5, en étant représenté, à la figure 14, implanté sur le côté postérieur des vertèbres 1 et 2. Ce dispositif 100_{inf} comporte ainsi des ensembles vertébraux supérieur 110_{inf} et inférieur 120_{inf}, respectivement identiques aux ensembles 110 et 120, en étant respectivement implantés sur les vertèbres 1 et 2 à l'aide de plots de fixation 112_{inf} et 122_{inf} respectivement identiques aux plots 112 et 122.

Le dispositif 100ₛᵤₚ présente, quant à lui, une structure analogue à celle du dispositif 100, mais inversée, dans le sens où son ensemble vertébral supérieur 110ₛᵤₚ est agencé postérieurement à son ensemble vertébral inférieur 120ₛᵤₚ. L'ensemble vertébral 110ₛᵤₚ est implanté sur une vertèbre 4 adjacente à la vertèbre 1 mais, à l'opposé de la vertèbre 2, située au-dessus de cette vertèbre 1, en en étant séparée par un disque intervertébral 5. A cet effet, l'ensemble vertébral 110ₛᵤₚ comporte deux plots de fixation osseuse 112ₛᵤₚ, analogues aux plots 112, dans le sens où ils délimitent intérieurement des orifices traversants de réception d'une vis d'ancrage osseux, qui débouchent sur la face postérieure 4P de la vertèbre 4.

L'ensemble vertébral inférieur 120ₛᵤₚ est implanté sur la vertèbre 1, par l'intermédiaire de plots de fixation osseuse 122ₛᵤₚ, analogues aux plots 122. Toutefois, comme bien visible sur les figures 13 et 14, ces plots 122ₛᵤₚ correspondent aux plots 112_{inf}, c'est-à-dire qu'ils sont constitués des mêmes pièces. Autrement dit, les dispositifs 100_{inf} et 100ₛᵤₚ ne sont pas indépendants l'un de l'autre, mais sont partiellement communs puisque leurs plots 112_{inf}/122ₛᵤₚ de fixation à la vertèbre 1 sont les mêmes.

De la sorte, l'ensemble de guidage 500 présente un encombrement moindre que la juxtaposition de deux dispositifs de guidage mécaniquement indépendants. En outre, les centres d'articulation C_{inf} et Cₛᵤₚ respectivement associés aux dispositifs 100_{inf} et 100ₛᵤₚ sont positionnés l'un par rapport à l'autre de manière fixe et prédéterminée, ce qui assure la maîtrise d'ensemble des cinématiques imposées aux ensembles vertébraux 110_{inf}, 120_{inf}, 110ₛᵤₚ et 120ₛᵤₚ par la coopération de leur surface d'articulation centrée sur ces centres. Le soulagement des disques 3 et 5 peut alors être contrôlé de manière efficace, notamment en étant uniformisé vis-à-vis de ces deux disques.

A titre de variantes, non représentées, de l'ensemble 500, l'un et/ou l'autre de ses deux dispositifs 100_{inf} et 100ₛᵤₚ peuvent être réalisés sous la forme des dispositifs 200, 300 et 400. De même, plus de deux dispositifs de guidage, du type des dispositifs 100, 200, 300 et 400, peuvent être intégrés à un même ensemble de guidage du type de l'ensemble 500, pour traiter quatre voire davantage de vertèbres adjacentes.

## Revendications

1. Dispositif (100 ; 200 ; 300 ; 400) de guidage dynamique postérieur du rachis, comportant deux ensembles vertébraux (110, 120 ; 210, 220 ; 310, 320 ; 410, 420) respectivement adaptés pour être fixés au côté postérieur de deux vertèbres adjacentes (1, 2) du rachis,
**caractérisé en ce que** les deux ensembles vertébraux (110, 120 ; 210, 220; 310, 320 ; 410, 420) définissent respectivement des surfaces d'articulation complémentaires (111 P, 121A ; 311₁A, 311₂A, 321₁P, 321₂P) sensiblement sphériques, qui, lorsque les deux ensembles vertébraux sont fixés aux vertèbres (1, 2), s'étendent globalement du côté postérieur des vertèbres et glissent l'une contre l'autre, en étant centrées en un même point géométrique (C) situé dans l'espace intervertébral séparant les deux vertèbres,
et **en ce qu'**il comporte des moyens mécaniques de limitation cinématique (130 ; 230 ; 330 ; 430), adaptés pour limiter l'amplitude des glissements relatifs des surfaces d'articulation (111P, 121A; 311₁A, 311₂A, 321₁P, 321₂P).

2. Dispositif suivant la revendication 1, **caractérisé en ce que** chaque ensemble vertébral (110, 120 ; 210, 220) comporte une unique coupelle (111, 121 ; 211, 221), délimitant toute la surface d'articulation correspondante (111P, 121A) sous la forme d'une calotte qui, lorsque l'ensemble vertébral est fixé à sa vertèbre associée (1, 2), s'étend de part et d'autre du plan sagittal de cette vertèbre.

3. Dispositif suivant la revendication 1, **caractérisé en ce que** chaque ensemble (310, 320 ; 410, 420) comporte une paire de deux coupelles distinctes (311₁, 311₂, 321₁, 321₂ ; 411₁, 411₂, 421₁, 421₂), distantes l'une de l'autre suivant une direction sensiblement médiolatérale et délimitant chacune pour partie, de préférence pour moitié, les surfaces d'articulation correspondantes (311₁A, 311₂A, 321₁P, 321₂P).

4. Dispositif suivant l'une des revendications 2 ou 3, **caractérisé en ce que** l'unique coupelle (111, 121 ; 211, 221) ou chaque coupelle (311₁, 311₂, 321₁, 321₂; 411₁, 411₂, 421₁, 421₂) de ladite paire de chaque ensemble vertébral (110, 120 ; 210, 220 ; 310, 320 ; 410, 420) a une concavité tournée vers le côté postérieur de la vertèbre associée (1, 2) lorsque l'ensemble vertébral est fixé à cette vertèbre.

5. Dispositif suivant l'une quelconque des revendications 2 à 4, **caractérisé en ce que** chaque ensemble vertébral (110, 120) comporte deux plots (112, 122) de fixation à la face postérieure (1P, 2P) de la vertèbre (1, 2) associée à cet ensemble, lesquels plots sont distants l'un de l'autre suivant une direction sensiblement médiolatérale et sont reliés tous les deux à l'unique coupelle (111, 121) ou à chaque coupelle de ladite paire par des pattes respectives (114, 124) formant chacune un prolongement périphérique de même courbure de la coupelle.

6. Dispositif suivant l'une quelconque des revendications 2 à 4, **caractérisé en ce que** chaque ensemble (210, 220 ; 310, 320) comporte deux plots (212, 222 ; 312, 322) de fixation osseuse à la face postérieure (1P, 2P) de la vertèbre (1, 2) associée à cet ensemble, lesquels plots sont distants l'un de l'autre suivant une direction sensiblement médiolatérale et sont reliés l'un à l'autre par une poutre de renfort (217, 227 ; 317, 327), à la fois munie des plots à chacune de ses extrémités longitudinales et reliées rigidement à l'unique coupelle (211, 221) ou à chaque coupelle (311₁, 311₂, 321₁, 321₂) de ladite paire.

7. Dispositif suivant l'une quelconque des revendications précédentes, **caractérisé en ce que** les moyens de limitation cinématique (130 ; 230 ; 330 ; 430) comportent, d'une part, un pion (135) lié cinématiquement à un premier (120) des deux ensembles vertébraux (110, 120) et s'étendant au moins partiellement en saillie de la ou d'une des surfaces d'articulation (121A) définie par ce premier ensemble vertébral, en direction de la ou d'une des surfaces d'articulation (111 P) définie par le second (110) des deux ensembles vertébraux, et, d'autre part, un orifice (115) de réception du pion avec des jeux de débattement (J), cet orifice étant ménagé par le second ensemble vertébral et débouchant sur la surface d'articulation définie par ce second ensemble vertébral.

8. Dispositif suivant la revendication 7, **caractérisé en ce que** l'orifice (115) traverse le second élément vertébral (110) de part en part suivant une direction sensiblement antéropostérieure et débouche ainsi sur une surface (111A) du second élément vertébral opposée à sa surface d'articulation (111P),
et **en ce que** le pion (135) s'étend dans cet orifice, en reliant de manière fixe, d'une part, une première pièce d'assemblage (132) agencée du côté de ladite surface opposée (111A) et, d'autre part, une seconde pièce d'assemblage (131) agencée du côté du premier ensemble vertébral (120) opposé à sa surface d'articulation (121 P).

9. Dispositif suivant la revendication 8, **caractérisé en ce que** ladite surface opposée (111A) du second ensemble vertébral (110) est sphérique de manière concentrique aux surfaces d'articulation (111P, 121A),
et **en ce que** la première pièce d'assemblage (132) délimite une surface sphérique (136P) complémentaire de ladite surface opposée (111A) et adaptée pour glisser contre cette surface opposée lors des glissements relatifs des surfaces d'articulation (111P, 121A).

10. Dispositif suivant l'une quelconque des revendications précédentes, **caractérisé en ce qu'**il comporte en outre un moyen d'amortissement élastique (150), interposé entre les deux ensembles vertébraux (110, 120).

11. Ensemble (500) de guidage dynamique postérieur du rachis, **caractérisé en ce qu'**il comporte au moins deux dispositifs (100_{inf}, 100ₛᵤₚ) conformes à l'une quelconque des revendications précédentes, et **en ce qu'**un premier ensemble vertébral (110_{inf}) de l'un (100_{inf}) des dispositifs et un premier ensemble vertébral (120ₛᵤₚ) de l'autre (100ₛᵤₚ) ou d'un des autres dispositifs sont partiellement communs, notamment pour ce qui concerne leur fixation osseuse, et adaptés pour être fixés à une même vertèbre intermédiaire (1), tandis que les seconds ensembles vertébraux (120_{inf}, 110ₛᵤₚ) des deux dispositifs sont respectivement adaptés pour être fixés à des vertèbres inférieure (2) et supérieure (4), adjacentes à la vertèbre intermédiaire.

12. Système de traitement du rachis, **caractérisé en ce qu'**il comprend, d'une part, un dispositif (100 ; 200 ; 300 ; 400) conforme à l'une quelconque des revendications 1 à 10, et, d'autre part, un moyen (140) de blocage temporaire de ce dispositif, adapté pour être relié de façon amovible aux ensembles vertébraux (110, 120) et pour bloquer alors les glissements relatifs des surfaces d'articulation (111P, 121A).

13. Système suivant la revendication 12, **caractérisé en ce que** le moyen de blocage (140) comporte deux broches (142) respectivement reçues dans des encoches complémentaires (116, 126) ménagées dans les ensembles vertébraux (110, 120), les deux encoches de chaque ensemble vertébral étant, à la fois, alignées dans une direction passant par la zone centrale de la ou d'une des surfaces d'articulation (111P, 121A) définie par cet ensemble vertébral et situées de part et d'autre de cette zone centrale suivant cette direction.
